# EUROPEAN PATENT SPECIFICATION

(11) **EP 2 221 007 B1**
(45) Date of publication and mention of the grant of the patent: **20.05.2015**
(21) Application number: 10002930.5
(22) Date of filing: 28.11.2000
(51) Int. Cl.: A61B 17/04

(54) **Wound closure device**
Vorrichtung zum Verschliessen einer Wunde
Dispositif de fermeture de plaie

(30) Priority: 02.12.1999 US 453120
(43) Date of publication of application: 25.08.2010
(62) Divisional of application: 00980833.8
(73) Proprietor: Smith & Nephew, Inc., Memphis, TN 38116 (US)
(72) Inventor: Torrie, Paul, Alexander, Marblehead, MA 01945 (US); Sikora, George, Mansfield, MA 02048 (US); Ek, Steven, Bolton, MA 01740 (US)
(74) Representative: Smith & Nephew

(56) References cited:
- EP-A- 0 598 219
- US-A- 3 910 281
- US-A- 4 235 238
- US-A- 4 741 330
- US-A- 4 823 794

## Description

The invention relates to wound closure devices.

Fibrous tissue wounds, such as muscle, ligament, and cartilage tears, can be repaired arthroscopically using sutures. Traditionally, to close a fibrous tissue wound, a surgeon would insert two suture needles into the tissue with sutures attached, thread the sutures across the wound, and then tie knots to fix the free ends of the sutures within the tissue.

To simplify the wound closure procedure and to improve fixation, various types of suture anchors have been developed. One example of a suture anchor is disclosed in Hayhurst, U.S. Patent No. 4,741,330. In Hayhurst, one end of a suture is fixed to a resiliently-deformable, bar-shaped suture anchor. The anchor is loaded into the bore of a hollow needle and deployed into or against the fibrous tissue. The surgeon then threads the suture across the wound and tensions a free end of the suture to pull the wound closed. When the surgeon tensions the suture, the bar in the anchor becomes oriented transversely to the suture hole, anchoring the suture in place.

Document EP-A-0 598 219 discloses the most relevant prior art.

The present invention is an improvement upon these prior art techniques and is defined in the attached claims. In general, an aspect of the invention features a wound closure device that includes a first anchor, a second anchor, and a flexible member that connects the first anchor to the second anchor. The flexible member is movably attached to the second anchor, such that pulling on a free end of the flexible member shortens a length of the flexible member between the first and second anchors.

Embodiments of the disclosure may include one or more of the following features.

The movable attachment of the flexible member to the second anchor allows the length of the flexible member between the first and second anchors to be shortened, but not lengthened.

The movable attachment includes a knot formed in the flexible member at the second anchor. The knot includes, e.g., a first portion of the flexible member that forms a loop, and a second portion (which might include the free end) that passes over a surface of the second anchor and through the loop. The surface can be, e.g., an exterior surface of the second anchor.

Pulling on the free end of the flexible member in one direction causes the flexible member to slide through the loop, but pulling on the flexible member in an opposite direction causes the loop to press the second portion against a compression surface of the second anchor, resisting increase in the length of the flexible member between the first and second anchors. The loop can be disposed within a partially enclosed region of the second anchor.

The second anchor can include a first section that defines a plurality of holes through which the first portion of the flexible member passes to form the loop. A second section of the second anchor defines a passage that connects to the partially enclosed region, and the free end of the suture passes through this passage.

The second anchor can include a rounded body and an appendage attached to the rounded body, where the rounded body and the appendage define a partially enclosed region therebetween. At least a portion of the flexible member passes through the partially enclosed region. In addition, the flexible member might wrap around the rounded body.

The rounded body can have a generally cylindrical shape, sized to fit within the bore of a hollow needle, and an axial groove. The groove extends across an axial length of the rounded body. The cross-sectional shape of the rounded body can be, e. g., a rectangle, an L, or a D.

The second anchor can also have a generally hemispherical shape, a generally crescent shape, or a T-shape. The crescent-shaped anchor can have sharp tip configured to penetrate tissue.

The second anchor defines a through-hole through which the flexible member passes. In addition, the device can also include a second flexible member that passes through the through-hole, where the second flexible member has a thickened portion that has a width greater than a width of the through-hole, such that the thickened portion cannot pass through the through-hole.

The first anchor includes a generally cylindrical body sized and shaped to fit within the bore of a hollow needle, and a projection, such as a longitudinal fin, extending from the cylindrical body. Alternatively, the first anchor can have a button shape. The first anchor can define a hole for passage of the flexible member therethrough, and the flexible member can be fixed to the first anchor.

In another aspect, the disclosure features a wound closure device that includes a first anchor; a second anchor; a flexible member connecting the first anchor to the second anchor, the flexible member having a free end and being movably attached to the second anchor, such that pulling on the free end of the flexible member shortens the length of the flexible member between the first and second anchors.

Embodiments of this aspect can include one or more of the following features.

The second anchor features a distal surface, a proximal surface, a generally cylindrical body and an appendage attached to the generally cylindrical body, there being a plurality of holes passing through the second anchor. The generally cylindrical body and the appendage can define an interior, partially enclosed region. The distal surface can be bevelled.

The appendage may be generally L-shaped in cross-section. The L-shape can be formed by a stem attached to the cylindrical body along an axial length of the body and a base attached to the stem such that it projects essentially perpendicularly thereto. The base may define one vertically transverse hole and stem may define two horizontally transverse holes directed essentially perpendicularly to the vertically transverse hole.

Alternatively, the appendage may include a stem. Two holes can be defined in the stem and a further hole can be defined essentially perpendicular thereto in the generally cylindrical body.

The appendage can be generally rectangular in cross section. The appendage can be disposed offset from the generally cylindrically body. The holes can be disposed through the appendage.

Alternatively, the appendage can be attached to the generally cylindrical body along the long side of the rectangle and can be centred along an axial length of the generally cylindrical body. The holes can be disposed through both the appendage and the generally cylindrical body.

In a further alternative embodiment, the appendage may comprise a stem attached to the generally cylindrical body and a head attached to the stem. The stem can be generally rectangular in cross section and the head generally D-shaped in cross section. The holes can be disposed through both the appendage and the generally cylindrical body.

The second anchor can have a generally hemispherical shape, a generally crescent shape or can be generally T-shaped. The second anchor can have a sharp tip configured to penetrate tissue.

The movable attachment of the flexible member to the second anchor enables the length of the flexible member between the first and second anchors to be shortened, but not lengthened. The moveable attachment includes a knot formed in the flexible member at the second anchor. The knot includes a first portion of the flexible member that forms a loop, and a second portion that passes over a surface of the second anchor and through the loop. The surface comprises an exterior surface of the second anchor.

The knot is configured such that pulling on the free end causes the flexible member to slide through the loop to shorten the length of the flexible member between the first and second anchors, but pulling on the flexible member in an opposite direction in order to increase the length of the flexible member between the first and second anchors causes the loop to press the second portion against a compression surface of the second anchor, resisting increase in the length of the flexible member between the first and second anchors.

The second anchor may define an interior, partially enclosed region and the loop is formed within the interior, partially enclosed region.

The second anchor can also include at least two loop-forming holes and the first portion of the flexible member can pass through the at least two loop-forming holes to form the loop. The second anchor can also include an additional hole that connects to the interior, partially enclosed region, and the free end of the suture can pass through the additional hole.

The first anchor can have a generally cylindrical body, the body being sized and shaped to fit within a bore of a hollow needle.

The first anchor can include a projection extending from the cylindrical body. The projection includes a longitudinal fin. The projection can define at least one hole for passage of the flexible member therethrough. The first anchor can have a button shape and defines at least one hole for the passage of suture therethrough.

The flexible member can be a suture.

In another aspect, the disclosure features a wound closure kit that includes an open-tipped needle and first and second anchors connected by a flexible member disposed within a bore of the needle.

Embodiments of this aspect of the disclosure may include one or more of the following features.

The needle can define a longitudinal slit, and the first anchor can include a projection that protrudes through the slit. The second anchor can also include an appendage that protrudes through the slit, and the flexible member can be attached to the appendage. The flexible member can be fixed to the first anchor, but movably attached to the second anchor.

In another aspect, the disclosure features a method of closing a tissue wound. The method includes: (a) providing a wound closure device that has a first anchor, a second anchor, and a flexible member movably attached to the second anchor; (b) positioning the first anchor against tissue; (c) passing the flexible member across the wound; (d) positioning the second anchor against tissue; and (e) pulling on a free end of the flexible member to shorten a length of the flexible member between the first and second anchors, thereby closing the wound.

This aspect of the disclosure may include one or more of the following features.

The flexible member can be slidably attached to the second anchor by a one-way knot, such that after completion of the pulling step, the length of the flexible member between the first and second anchors remains shortened. Part of the one-way knot can be disposed within a partially enclosed region of the second anchor. The partially enclosed region can be located such that during the pulling step, the portion of the one-way knot disposed within the partially enclosed region avoids contact with tissue.

The first positioning step can include positioning the first anchor on a first side of the wound. The passing step can include passing the flexible member from the first side to a second side of the wound, engaging tissue on the second side, and returning the flexible member to the first side. The second positioning step can include positioning the second anchor on the first side of the wound.

The first and second anchors can initially be disposed, at least partially, within the bore of a hollow, open-tipped needle. The first anchor can include a section that protrudes through a slit in the needle, and the first positioning step can include engaging the projection with tissue to remove the first anchor from the bore.

The providing step can include providing a device in which the flexible member passes through a through-hole in the second anchor, and the device further includes a second flexible member that also passes through the through-hole. The second flexible member has a thickened portion that is wider than the through hole. The method further includes pulling the second flexible member until the thickened portion wedges into the through hole.

In another aspect, the disclosure features a method of repairing a tear in a meniscus. The method includes: (a) providing a tear closing device having a first anchor, a second anchor, and a flexible member movably attached to the second anchor; (b) positioning the first anchor against the meniscus; (c) passing the flexible member across the tear; (d) positioning the second anchor against the meniscus; and (e) pulling on a free end of the flexible member to shorten a length of the flexible member between the first and second anchors, thereby closing the tear.

Embodiments of this aspect of the disclosure can include one or more of the following features.

The first positioning step can include positioning the first anchor against an external surface of the meniscus, on a first side of the tear, the passing step can include passing the flexible member from the first side to a second side of the tear, engaging tissue on the second side, and returning the flexible member to the first side, and the second positioning step can include positioning the second anchor against the external surface. Alternatively, the first and second anchors can be positioned against external surfaces of the meniscus on opposite sides of the tear.

Embodiments of the disclosure may include one or more of the following advantages. The first and second anchors can be deployed using a single hollow needle, rather than two separate needles. After deploying the anchors, the surgeon need not tie an additional knot. The length of the flexible member between the two anchors can be adjusted after deploying the anchors, allowing a surgeon to set the tension in the flexible member to a desired level.

Since the device uses a flexible member, such as a suture, to close the tissue wound, rather than inflexible staples or tacks, the tissue is not significantly damaged when it expands and contracts. For example, if the soft tissue is a meniscus, the anchors do not damage the meniscal tissue when the knee moves.

Other features and advantages disclosure will become apparent from the following detailed description and from the claims.
Fig. 1A is a perspective view of a wound closure device.
Fig. 1B is a side view of a first suture anchor of the wound closure device of Fig. 1A.
Fig. 1C is a side view of a second suture anchor of the device of Fig. 1A.
Fig. 1D is an enlarged view of a braided suture.
Fig. 2 is a perspective, cut-away view of the wound closure device of Fig. 1A implanted within a meniscus.
Figs. 3A and 3B are sectional views of the second suture anchor and one-way knot of the wound closure device of Fig. 1A.
Fig. 4 is a perspective view of a hollow needle, with the wound closure device of Fig. 1A disposed within a bore of the needle.
Figs. 5A-5D are schematics illustrating deployment of the wound closure device of Fig. 1A into a meniscus using a plunger.
Figs. 6A-6D are schematics illustrating deployment of the wound closure device of Fig. 1A into a meniscus using a plunger and a spacer.
Fig. 7A is a perspective view of an alternative first anchor design for a wound closure device, used in reverse deployment of the device.
Figs. 7B-7E are schematics illustrating reverse deployment of a wound closure device.
Figs. 8A and 8B are perspective views of the second suture anchor of Figs. 1A and 1C, showing an alternative one-way knot configuration for the anchor.
Figs. 9-12 are sectional views of alternative configurations of the suture anchor and one-way knot of Figs. 3A and 3B.
Fig. 13A is a top view of a T-shaped second suture anchor.
Fig. 13B is a side view of the T-shaped suture anchor of Fig. 13A.
Fig. 13C is a front view of a crescent-shaped second suture anchor.
Fig. 13D is a side view of the crescent-shaped suture anchor of Fig. 13C.
Figs. 14-16 are sectional views showing deployment of wound closure devices that include the crescent-shaped suture anchor of Fig. 13C.
Fig. 17 is a perspective, partially schematic view of an alternative wound closure device implanted within a meniscus.
Fig. 18A is a perspective view of an alternative first suture anchor design for a wound closure device.
Fig. 18B is a perspective, cut-away view of the wound closure device of Fig. 18A implanted within a meniscus.

Embodiments of the present invention feature wound closure devices that include two suture anchors connected by a length of a flexible member, such as a suture. The suture is tied to the first anchor with a conventional knot, but movably attached to the second anchor, allowing a surgeon to shorten the length of suture between the anchors, and thereby close a wound. In some embodiments, the two anchors can be deployed within torn tissue using a single hollow needle.

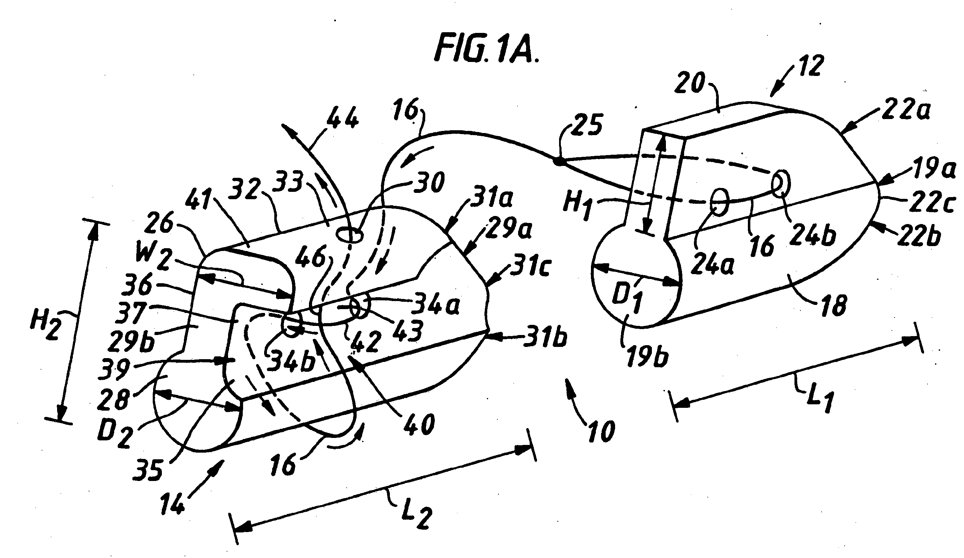

Referring to Figs. 1A-1C, a wound closure device 10 includes a first suture anchor 12, a second suture anchor 14, and a suture 16 that connects anchor 12 to anchor 14. First anchor 12 has a generally solid cylindrical body 18 extending axially from a distal surface 19a to a flat proximal surface 19b. To facilitate passage of first anchor 12 into tissue, both an upper portion 22a and a lower portion 22b of surface 19a are beveled relative to the axis of cylindrical body 18, forming a rounded distal tip 22c. Upper portion 22a is beveled at an angle á relative to the axis of cylindrical body 18, and lower portion 22b is beveled at an angle a relative to the axis.

Attached to cylindrical body 18 is a fin-shaped projection 20 that extends from upper portion 22a of distal surface 19a to proximal surface 19b. Fin 20 defines two horizontally transverse holes, 24a and 24b. Suture 16 is attached to first anchor 12 by passing the suture through hole 24a in a first direction (e.g., out of the page in Fig. 1A), through hole 24b in a second direction (e.g., into the page in Fig. 1A), and then forming a conventional knot 25 near fin 20. Conventional knot 25 rigidly fixes suture 16 to the first anchor.

Second anchor 14 has a distal surface 29a, a proximal surface 29b, and a generally cylindrical body 28 extending axially from surface 29a to surface 29b. Attached to body 28 is an appendage 26. Appendage 26 is generally L-shaped in cross-section, and extends along an axial length of body 28, from surface 29a to surface 29b.

Distal surface 29a of anchor 14 is beveled in a manner similar to anchor 12: An upper portion 31a of surface 29a forms an angle È relative to an axis of body 28, and a lower portion 31b of surface 29a forms an angle ö relative to the body's axis, forming a rounded distal tip 31c. Proximal surface 29b of anchor 14 is flat.

The L-shape of appendage 26 is formed by two perpendicular sections: a stem 36 attached to cylindrical body 28 along an axial length of the body, and a base 32 attached to the stem. Base 32 defines a vertically transverse hole 30, and stem 36 defines two horizontally transverse holes 34a and 34b. Holes 34a and 34b are perpendicular to hole 30, but all three holes pass through anchor 14 in a direction generally parallel to sides 29a and 29b.

Each of the three holes 34a, 34b, and 30 connect the exterior of anchor 14 to an interior, partially enclosed, protected region 39. Region 39 is defined by base 32, an interior surface 37 of stem 36, and a concave groove 35 within cylindrical body 28. Groove 35 extends axially across body 28, along the line where interior surface 37 connects to body 28.

Suture 16 is movably attached to second anchor 14 with a one-way knot 40. One-way knot 40 includes a first portion of suture 16 that forms a loop 42, and a second portion of suture that passes around body 28 and through the loop. Loop 42 is formed within the protective region 39 defined by groove 35, surface 37, and base 32. Region 39 acts to separate loop 42 from tissue when device 10 is implanted within tissue, preventing the tissue from interfering with the sliding action of one-way knot 40.

To form one-way knot 40, suture 16 is first passed from an exterior of anchor 14, through hole 34a into region 39, and then back out hole 34b to the exterior, forming loop 42 within region 39. The suture is then passed over the rounded, exterior surface 41 of cylindrical body 28, back into region 39, through loop 42, and then to the exterior through hole 30. Suture 16 terminates at a free end 44.

One-way knot 40 allows the length of suture between first anchor 12 and second anchor 14 to be shortened, but not lengthened. A surgeon can shorten the length of suture between the anchors by pulling on free end 44, which draws additional suture in the direction of the arrows in Fig. 1A, through holes 30, 34b, and 34a, thereby reducing the length of suture between anchors 12 and 14. If, however, the surgeon attempts to lengthen the distance between the anchors, e.g., by pulling first anchor 12 away from second anchor 14 (i.e., pulling suture 16 in the opposite direction of the arrows), then loop 42 squeezes portion 46 of suture 16 against an interior compression surface 43 of stem 36, preventing further lengthening of the distance between the anchors.

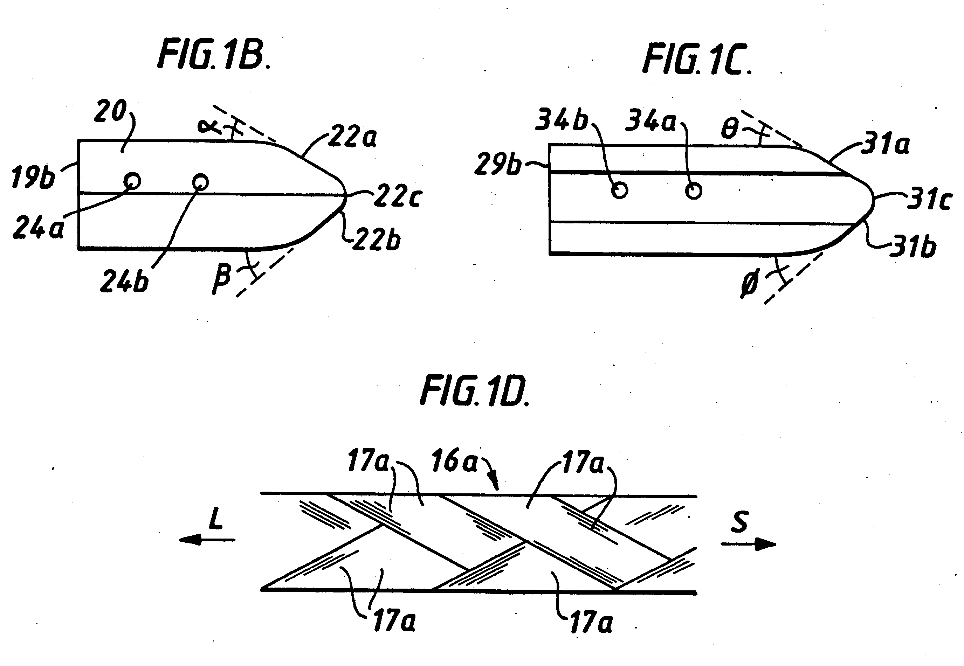

If suture 16 is a braided suture, as opposed to a smooth suture, then suture 16 should be threaded through second anchor 14 in a particular direction. Referring to Fig. 1D, a braided suture 16a is formed from numerous threads 17a braided from left to right in Fig. 1D. Braided suture 16a slides more easily if it is pulled in the direction of braiding (i.e., in the direction of arrow S in Fig. 1D) than if it is pulled against the braiding (in the direction of arrow L). Thus, if suture 16 is a braided suture, then suture 16 should be threaded through second anchor 14 in the direction of braiding. If threaded in the direction of braiding, the suture will slide more easily in the direction of the arrows in Fig. 1A, and less easily in the direction opposing the arrows.

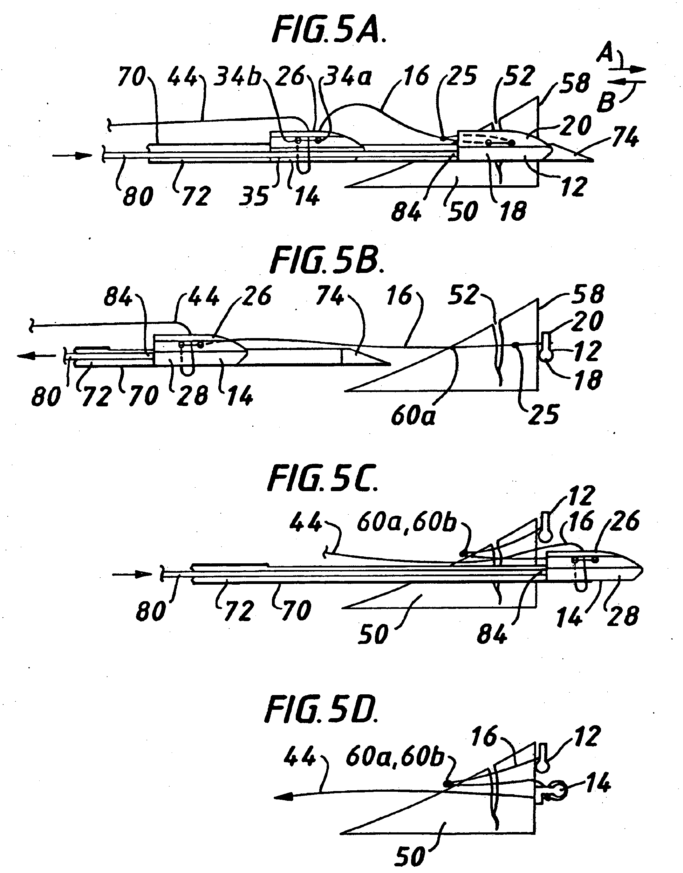

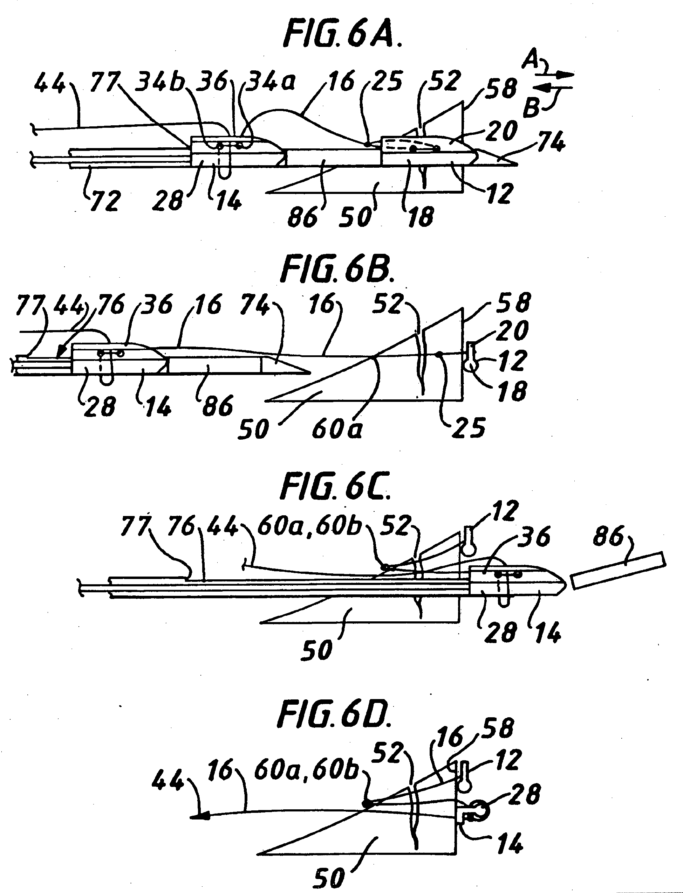

The cylindrical portions of anchors 12 and 14 are sized and shaped to fit within a hollow bore of a needle (described below), facilitating arthroscopic implantation of device 10. For example, cylindrical body 18 has a diameter D₁ of about 0.04 inches, and cylindrical body 28 has a diameter D₂ approximately equal to diameter D₁. Fin-shaped projection 20 and L-shaped appendage 26, however, are configured to protrude through a longitudinal slit in the needle. Delivery of device 10 using a hollow needle is described below, with reference to Figs. 5A-5D and 6A-6D.

First anchor 12 has an overall axial length L₁ of, e.g., about 0.19 inches, and fin 20 has a height H₁ of, e.g., about 0.03 inches. Second anchor 14 has an overall axial length L₂ of, e.g., about 0.22 inches, a width W₂ of, e.g., about 0.06 inches, and a height H₂ of, e.g., about 0.07 inches. Angle á is, e.g., about 30 degrees, angle â is, e.g., about 40 degrees, angle È is, e.g., about 30 degrees, and angle ö is, e.g., about 40 degrees.

Anchors 12 and 14 are made from rigid, biocompatible materials, such as polyethylene, an acetal, or polypropylene. Alternatively, the anchors can be made from resiliently deformable materials, as described in Hayhurst, *supra*, or from bioabsorbable materials. Anchors 12 and 14 are preferably unitary, injection molded pieces, but can also be manufactured by other methods.

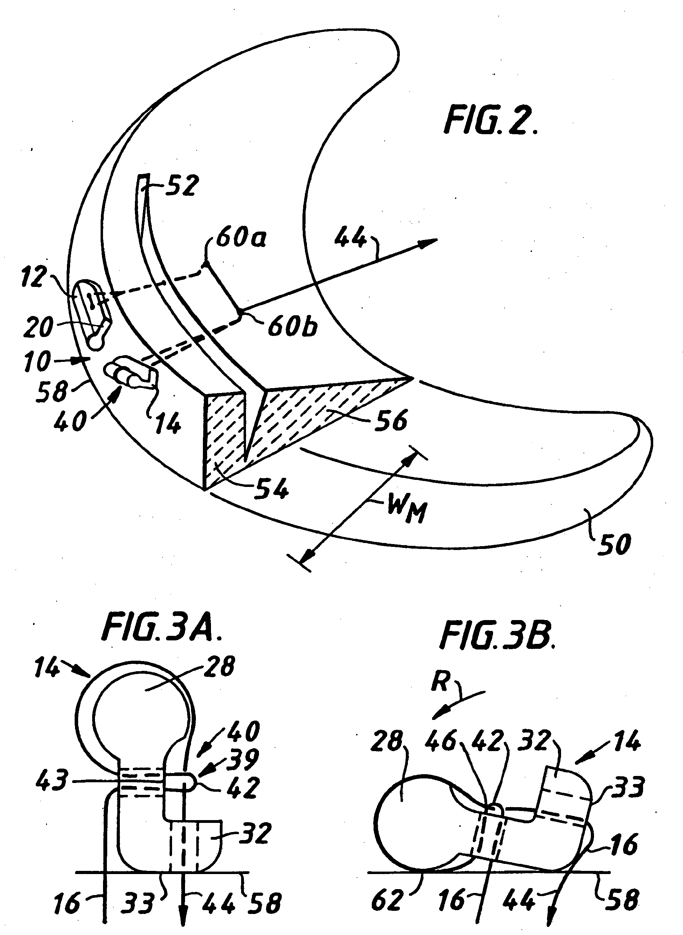

Fig. 2 illustrates the use of wound closure device 10 to repair a torn meniscus 50. Meniscus 50 is a C-shaped, rubbery, shock-absorbing structure located between the tibia and femur inside the knee. Meniscus 50 has a tear 52 that unnaturally separates distal meniscal tissue 54 from proximal tissue 56. A width W_{M} of meniscus 50, as measured from points 60a and 60b to an exterior surface 58 of the meniscus is, e.g., about 0.25 inches.

When device 10 is implanted within meniscus 50, both anchors 12 and 14 abut surface 58, separated by a distance of, e.g., about 1 cm. Suture 16 passes from first anchor 12, into distal tissue 54, across tear 52, and emerges from proximal tissue 56 at point 60a. Suture 16 then passes again into proximal tissue 56 at point 60b, again traverses tear 52, and emerges out surface 58, where it attaches to second anchor 14 via one-way knot 40. From second anchor 40, suture 16 passes again into distal tissue 54, traverses tear 52, and emerges from proximal tissue 56 at or near point 60b. Free end 44 of suture 16 emerges from proximal tissue 56.

Referring to Figs. 2, 3A, and 3B, once device 10 is implanted, a surgeon can close tear 52 by pulling on free end 44 of suture 16. When the surgeon pulls on free end 44, four separate movements occur in succession. First, friction between suture 16 and body 28 rotates second anchor 14 until a lower surface 33 of base 32 is flush against meniscal surface 58, as shown in Fig. 3A. Second, tension in suture 16 pulls the center of fin 20 towards surface 58, causing first anchor 12 to align against surface 58 transversely to the portion of suture 16 that exits anchor 12, with both fin 20 and the axial length of body 18 pressing against meniscal surface 58. Third, continued pulling on free end 44 draws additional suture through holes 30, 34b, and 34a, via knot 40, in the direction of the arrows of Fig. 1A, lengthening free end 44 and shortening the length of suture between anchors 12 and 14. Shortening the length of suture between anchors 12 and 14 increases the tension in suture 16 between the anchors, which pulls distal tissue 54 and proximal tissue 56 together, closing tear 52. Since loop 42 remains within protected region 39 as the surgeon pulls on free end 44, base 32 separates loop 42 from tissue, and suture 16 does not become wedged between tissue and anchor 14 when the surgeon pulls on the suture's free end. Once tear 52 has been closed, one-way knot 40 prevents the two anchors from pulling apart, and prevents the tear from re-opening.

The final successive movement occurs when the surgeon releases free end 44, after closing tear 52. When the surgeon releases the free end, the tension in suture 16 between the two anchors pulls body 28 of anchor 14 away from free end 44, causing second anchor 14 to rotate in the direction of arrow R (Fig. 3B), until body 28 abuts meniscal surface 58, trapping a portion 62 of suture 16 between body 28 and surface 58. (For clarity, suture 16 is shown spaced slightly from body 28 and stem 36 in Fig. 3B. In actuality, suture 16 is flush against the surfaces of anchor 14 after suture 16 is tensioned by the surgeon.)

When second anchor 14 is in its final position, shown in Fig. 3B, suture 16 is locked in place. The length of suture between anchors 12 and 14 cannot be increased, because loop 42 of one-way knot 40 presses portion 46 of the suture against surface 43 of stem 36. In addition, the length of suture between the anchors resists being further shortened, since portion 62 of suture 16 is wedged between body 28 and surface 58 of the meniscus.

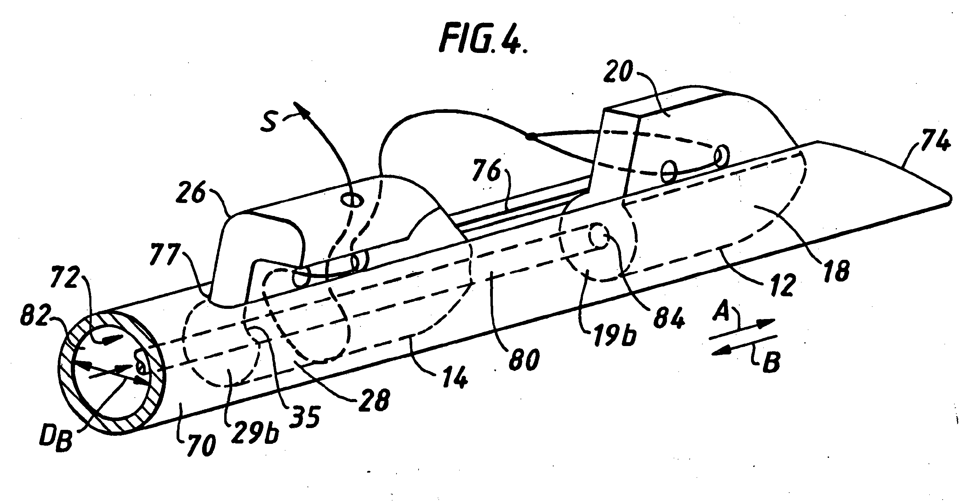

Wound closure device 10 is preferably deployed within meniscus 50 arthroscopically, using a hollow needle 70. Referring to Fig. 4, hollow needle 70 defines a bore 72 and an open distal tip 74. The diameter D_{B} of bore 72 is slightly larger than diameter D₁ of body 18 of first anchor 12, and diameter D₂ of body 28 of second anchor 14, allowing body 18 and body 28 to fit slidably within the bore. Needle 70 also includes a longitudinal slit 76 through a wall of the needle. Slit 76 extends proximally from open tip 74, and communicates with bore 72. Slit 76 is sized and shaped to allow fin 20 of first anchor 12 and L-shaped appendage 26 of second anchor 14 to protrude from needle 70.

Needle 70 also includes a plunger 80. Plunger 80 enters bore 72 through a proximal opening 82 in needle 70, and extends to proximal surface 19b of first anchor 12. Plunger 80 passes by second anchor 14 by sliding along groove 35. When plunger 80 is positioned as shown in Fig. 4, sliding plunger 80 in the direction of arrow A pushes first anchor 12 distally, but does not move second anchor 14.

Prior to surgery, suture 16 is attached to anchors 12 and 14, in the manner described above with reference to Fig. 1A. The two anchors 12 and 14 are then loaded into bore 72 of needle 70. Second anchor 14 is loaded first, by inserting cylindrical head 28 into bore 72, through open tip 74, such that appendage 26 protrudes through slit 76. Second anchor 14 is pushed proximally into slit 76, until stem 36 abuts a proximal surface 77 of the slit. Next, first anchor 12 is loaded into the distal most position in needle 70 by inserting cylindrical body 18 through tip 74, into bore 72, such that fin 20 protrudes through slit 76. Both anchors 12 and 14 are loaded with their respective beveled distal surfaces 19a and 29a facing open distal tip 74.

After the anchors have been loaded, plunger 80 is inserted into bore 72 through proximal opening 82. Plunger 80 is slid past second anchor 14 along groove 35, until a tip 84 of the plunger abuts proximal surface 19b of first anchor 12.

Attachment of suture 16 to anchors 12 and 14 and loading of the anchors and plunger into needle 70 can be performed at the time of manufacture, i.e., pre-loaded, or immediately prior to surgery.

During surgery (or prior to surgery), the surgeon first pushes plunger 80 in the direction of arrow A to separate anchors 12 and 14 within bore 72. The surgeon pushes the plunger until the anchors are separated by at least a distance L, as shown in Fig. 4, where L is greater than width W_{M} of meniscus 50. Distance L is, e.g., about 0.35 inches.

Referring to Figs. 2 and 5A-5D (not to scale), the surgeon next pushes needle 70 through meniscus 50, in the direction of arrow A, until fin 20 of first anchor 12 passes entirely through exterior surface 58 of the meniscus. As the surgeon pushes needle 70 through the tissue, he or she holds plunger 80 steady, to prevent anchor 12 from sliding in the direction of arrow B as the needle is pushed through the meniscal tissue. Since the separation distance L is greater than the width W_{M} of meniscus 50, second anchor 14 does not enter the meniscus at this point in the procedure.

The surgeon next forces first anchor 12 out of needle 70 through tip 74 by pushing plunger 80 in the direction of arrow A, and then seats anchor 12 against surface 58 of the meniscus by pulling on free end 44 of suture 16. Once anchor 12 has been seated, needle 70 is pulled in the direction of arrow B, back through meniscus 50, across tear 52, and out the hole at point 60a (Fig. 5B).

The surgeon then reinserts needle 70 into meniscus 50 at point 60b, and again passes the needle through the meniscus in the direction of arrow A, across tear 52, until tip 74 passes through surface 58. To eject second anchor 14, the surgeon withdraws plunger 80 in the direction of arrow B until tip 84 of the plunger is proximal to surface 29b of anchor 14. The surgeon then maneuvers plunger 80 until tip 84 contacts surface 29b, and then pushes the plunger in the direction of arrow A, forcing second anchor 14 through tip 74. Plunger 80 and needle 70 are then fully withdrawn in the direction of arrow B, leaving both anchors 12 and 14 resting against surface 58, as shown in Fig. 5D. The surgeon can then tension suture 16, positioning the anchors against surface 58 and closing tear 52, by pulling on free end 44, as described above with reference to Fig. 2. Other embodiments are within the scope of the claims. For example, device 10 can be extracted from needle 70 by engaging fin 20 with surface 58, rather than by using a plunger 80. Referring to Fig. 6A-6D (not to scale), in this embodiment, plunger 80 is not passed through groove 35 to first anchor 12. Instead, tip 84 of plunger 80 always remains proximal to anchor 14.

When the two anchors are loaded into bore 72 of needle 70, a spacer 86 is placed between the anchors. Spacer 86 is a simple cylindrical bar, preferably made from a material that degrades quickly in the body, such as salt. Spacer 86 has a longitudinal length L_{S} greater than width W_{M} of meniscus 50. Length L_{S} is, e.g., about 0.35 inches.

During surgery, the surgeon first pushes needle 70 through meniscus 50, in the direction of arrow A, until fin 20 passes entirely through exterior surface 58 of the meniscus (Fig. 6A). As the surgeon pushes needle 70 through the tissue, surface 77 of slit 76 engages stem 36 of anchor 14, preventing the two anchors and the spacer from sliding in the direction of arrow B within bore 72. (In addition, the surgeon can hold plunger 80 steady to prevent the two anchors from sliding in the direction of arrow B.)

The surgeon next pulls the needle in the direction of arrow B, back through meniscus 50, across tear 52, and out the hole at point 60a (Fig. 6B). As the surgeon withdraws the needle, fin 20 engages surface 58, and first anchor 12 is pulled out of needle 70, through tip 74. As before, the surgeon then seats anchor 12 against surface 58 by pulling on free end 44 of suture 16. Since spacer 86 is larger than width W_{M} of meniscus 50, the spacer prevents second anchor 14 from entering meniscus 50, and therefore prevents second anchor 14 from also being pulled out of needle 70 as needle 70 is pulled in the direction of arrow B.

Next, the surgeon reinserts needle 70 into meniscus 50 at point 60b, and again passes the needle through the meniscus in the direction of arrow A, across tear 52, until tip 74 passes through surface 58. The surgeon then pushes plunger 80 in the direction of arrow A, ejecting both spacer 86 and second anchor 14 out of needle 70 through tip 74 (Fig. 6C). The needle is then fully withdrawn from meniscus 50, in the direction of arrow B, leaving both anchors 12 and 14 resting against surface 58, as shown in Fig. 6D. The surgeon then pulls on free end 44 to position the anchors and close the tear, as described above. Spacer 86 can either be removed by the surgeon, or left within the body to degrade.

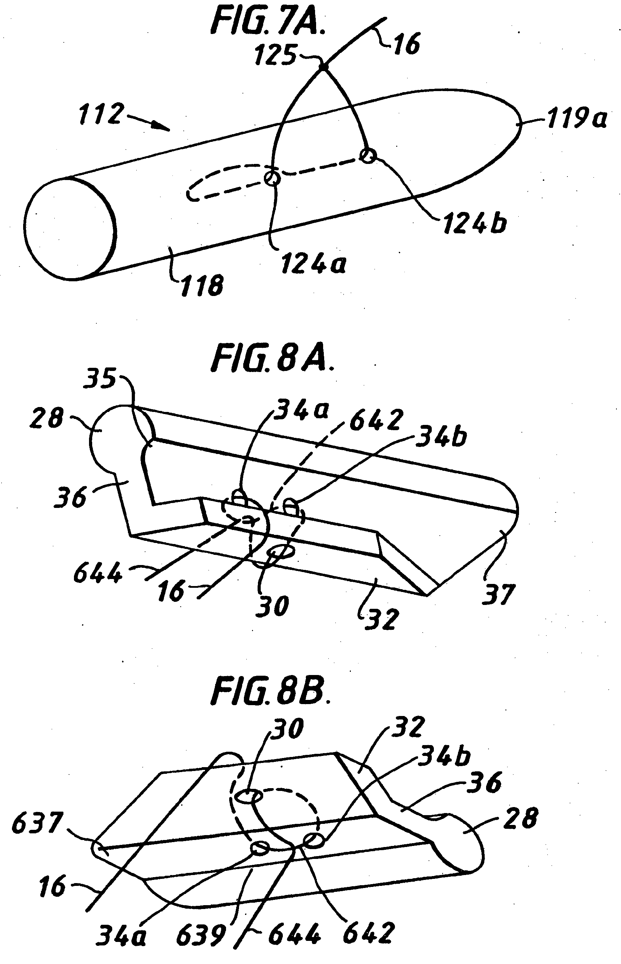

In a second alternative deployment method, a modified wound closure device is deployed in meniscus 50 in reverse, second anchor first. Referring to Fig. 7A, a modified wound closure device includes a first anchor 112 that has a beveled face 119a, but lacks a fin. Anchor 112 has an axial, generally cylindrical body 118 that defines two transverse holes 124a and 124b. Suture 16 is attached to anchor 112 by threading the suture through hole 124a in a first direction, through hole 124b in a second direction, and then tying a conventional knot 125.

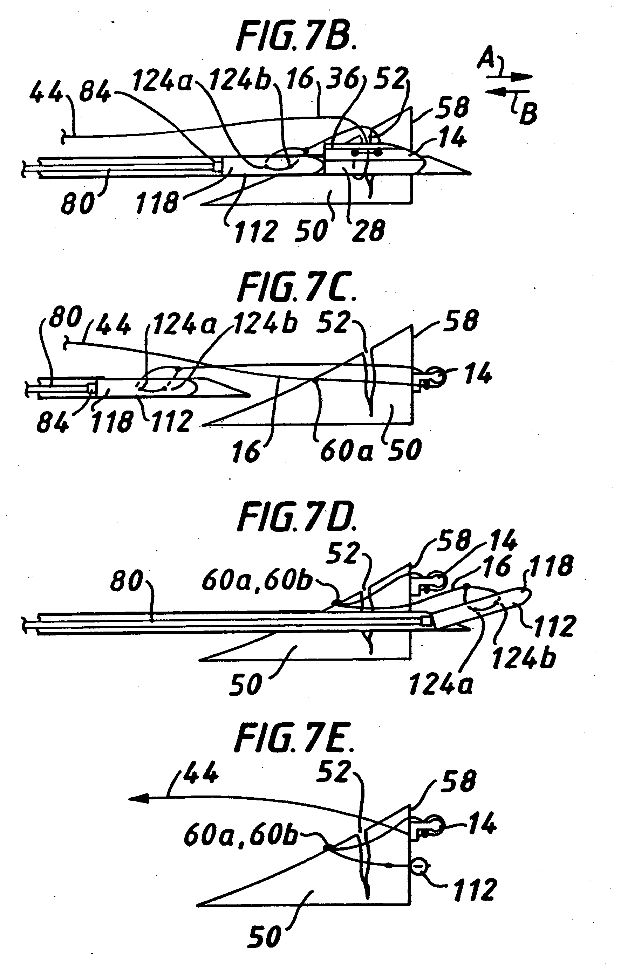

Referring to Figs. 7B-7E (not to scale), in this reverse deployment embodiment, second anchor 14 is positioned distally in bore 72, with first anchor 112 directly proximal. Tip 84 of plunger 80 resides immediately proximal to first anchor 112 in bore 72. In operation, the surgeon first pushes needle 70 through meniscus 50, in the direction of arrow A, until tip 74 passes through surface 58. The surgeon then pushes plunger 80 in the direction of arrow A far enough to force second anchor 14 through tip 74, but not far enough to eject first anchor 112.

After anchor 14 has been ejected, the surgeon pulls the needle in the direction of arrow B, back through meniscus 50, across tear 52, and out point 60a (Fig. 7C). Since first anchor 112 does not include a fin, it does not protrude through slit 76, and does not engage tissue when the needle is pulled in the direction of arrow B. Next, the surgeon reinserts needle 70 into the meniscus at point 60b, and again passes the needle through meniscus 50 in the direction of arrow A, across tear 52, until tip 74 passes through surface 58. The surgeon then ejects first anchor 112 by pushing plunger 80 in the direction of arrow A, (Fig. 7D), and withdraws needle 70 from meniscus 50. The surgeon then positions the anchors and closes tear 52 by pulling on free end 44 (Fig. 7E), as described above.

Alternative configurations of second anchor 14 and one-way knot 40 are possible.

For example, referring first to Figs. 8A and 8B, suture 16 can be threaded through second anchor 14 so that the loop is located against an exterior surface 637 of stem 36, rather than within region 39. In this embodiment, suture 16 is threaded through second anchor 14 by first passing the suture around base 32, into region 39, and then out of region 39 through hole 34a. The suture then passes back into region 39 through hole 34b, forming a loop 642 adjacent surface 637. After forming the loop, the suture passes through hole 30 to the exterior, and then through loop 642, terminating at free end 644.

When the surgeon pulls free end 644 of suture 16, the anchor rotates until surface 637 faces surface 58 of meniscus 50 (Figs. 2 and 3A-3B). Cylindrical body 28 causes part of surface 637 to remain elevated above surface 58, creating a small gap 639 that contains loop 642. Loop 642, therefore, does not become wedged between tissue and surface 637 when the surgeon pulls free end 644 to tension the suture.

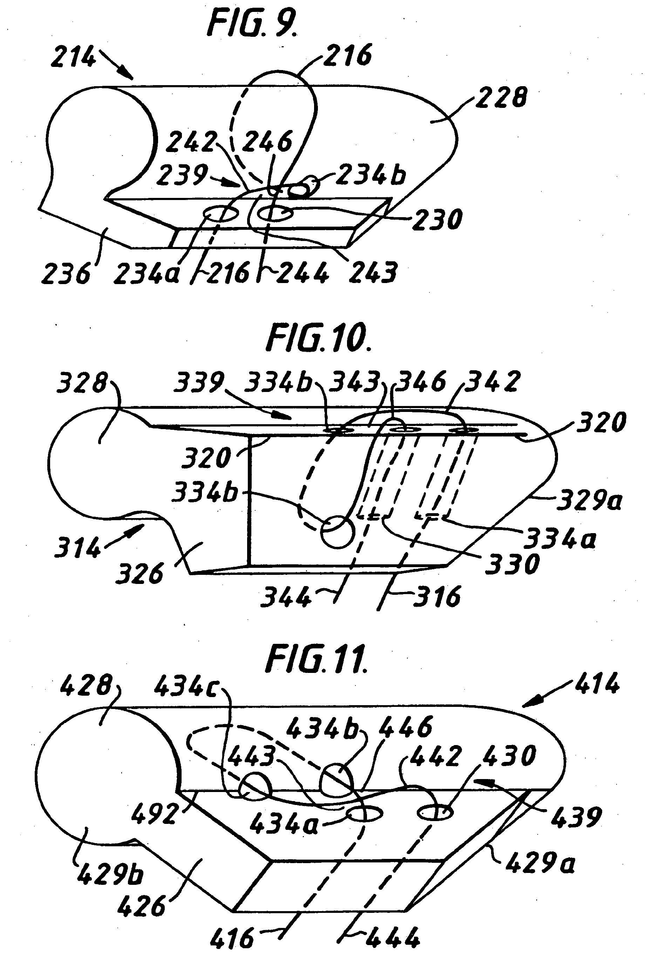

Referring to Fig. 9, second anchor 214 has a structure similar to anchor 14, except that anchor 214 lacks a base 32. Anchor 214 includes a cylindrical body 228 and a stem 236 that define a wedge-shaped, partially enclosed region 239 therebetween. Stem 236 defines two holes, 234a and 230, and body 228 defines one transverse hole 234b. Holes 234a and 230 are generally parallel, and are both generally perpendicular to hole 234b. A suture 216 passes from a first anchor (not shown) through hole 234a into region 239, and then through hole 234b to the exterior of the anchor, forming a loop 242 within region 239. The suture then wraps around body 228 back into region 239, through loop 242, and out of region 239 through hole 230, terminating at free end 244. Alternatively, suture 216 can wrap around body 228 two or more times before passing back into region 239.

As in the previous embodiments, pulling on free end 244 tensions suture 216 and shortens the length of suture between the anchors. Pulling on suture 216 in an opposite direction, however, causes loop 242 to press a portion 246 of the suture against a compression surface 243. Since loop 242 is located within protected region 239, and is therefore spaced from the meniscal surface, loop 242 does not become wedged between tissue and the anchor when the surgeon tensions suture 216. Unlike the previous embodiments, however, anchor 214 does not rotate after the surgeon tensions the suture and releases free end 244.

Referring to Fig. 10, second anchor 314 includes a generally cylindrical body 328 and an offset, generally rectangular appendage 326. Appendage 326 and body 328 define a partially protected region 339. As with anchor 14, a front surface 329a of anchor 314 is beveled.

Appendage 326 defines three holes, 330, 334a, and 334b. Holes 334a and 330 are generally straight, while hole 334b defines an arc through an inside of appendage 326. A suture 316 passes from a first anchor (not shown) through straight hole 334a into region 339. Suture 316 then passes out of region 339 through arc-shaped hole 334b, forming a loop 342 within region 339. The suture then wraps around a corner 320 of body 326, passes through loop 342, and through hole 330, terminating at free end 344. As with the embodiments of Figs. 1, 7A-7B, and 8, pulling on free end 344 shortens the length of suture between the anchors, but pulling on suture 316 in an opposite direction causes loop 342 to squeeze a portion 346 of the suture against a compression surface 343 of appendage 326, preventing further movement. Like the embodiment of Fig. 8, anchor 314 does not rotate after the suture is tensioned and released.

Referring to Fig. 11, second anchor 414 includes a generally cylindrical body 428 extending from a beveled distal surface 429a to a flat proximal surface 429b. A generally rectangular appendage 426 also extends from surface 429a to surface 429b. Rectangular appendage 426 is attached to body 428 along the long side of the rectangle, and is centered along an axial length of body 428. Body 428 and appendage 426 define a protected region 439.

Appendage 426 defines two holes, 434a and 430, and body 428 defines two transverse holes, 434b and 434c. Hole 434b is located entirely within body 428, but hole 434c is located at a juncture 492 between body 428 and appendage 426. Holes 434a and 430 are generally parallel, and are both generally perpendicular to holes 434b and 434c.

A suture 416 extends from a first anchor (not shown), through hole 434a into region 439, and then out of region 439 through hole 434b, forming a loop 442 within region 439. The suture then passes back into region 439 through hole 434c, through loop 442, and out of region 439 through hole 430, terminating at free end 444.

As with the other described second anchor embodiments, pulling on free end 444 tensions suture 416 and shortens the length of suture between the anchors, but pulling on suture 416 in an opposite direction causes loop 442 to squeeze a portion 446 of the suture against a compression surface 443 of appendage 426, preventing further movement. Region 439 is separated from the meniscal tissue by body 428, preventing loop 442 from wedging between anchor 414 and tissue when the surgeon pulls on free end 444. Anchor 414 does not rotate after the surgeon tensions and releases the suture.

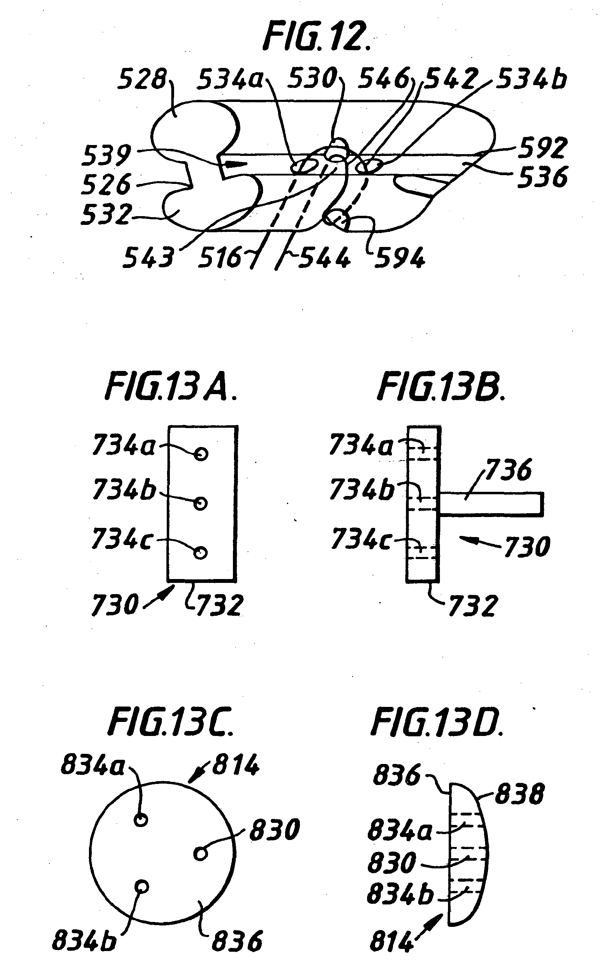

Referring to Fig. 12, second anchor 514 includes a generally cylindrical body 528 and an appendage 526. Both body 528 and appendage 526 extend from a beveled distal surface 529a to a flat proximal surface 529b. Appendage 526 includes a stem 536 attached to body 528, and a head 532 attached to stem 536. Stem 536 is rectangular in cross-section and head 532 is D-shaped in cross-section, giving appendage 526 a mushroom-shaped cross-section. Stem 536, head 532, and body 528 define a partially enclosed, protected region 539.

Stem 536 defines two transverse holes 534a and 534b, and body 528 defines a single transverse hole 530 located at a juncture 592 between body 528 and stem 536. A suture 516 passes from a first anchor (not shown), through hole 534a into protected region 539, and then out of region 539 through hole 534b, forming a loop 542 within region 539. Suture 516 then passes around D-shaped head 532 through a transverse groove 594 in head 532, back into region 539, through loop 542, and out hole 530, terminating at free end 544. As with the other described embodiments of the second anchor, pulling on free end 544 shortens the distance between the two anchors, but pulling on suture 516 in an opposite direction causes loop 542 to squeeze a portion 546 of the suture against a compression surface 543 of stem 536, preventing further movement. That loop 542 is located within region 539 prevents the loop from becoming wedged between the anchor and tissue when the surgeon pulls on free end 544. Anchor 514, like anchors 214, 314, and 414, does not rotate after the surgeon tensions and releases the suture.

Referring to Figs. 13A-13D and Figs. 14-16, the second anchor can have a T shape or a crescent shape, and can be deployed at a surface of the meniscus or inter-body. Referring to Figs. 13A and 13B, second anchor 730 has a T-shape. The base 732 of the T defines three through-holes, 734a, 734b, and 734c, and the stem 736 of the T is configured to penetrate meniscal tissue. Stem 736 is offset from base 732 so that the stem does not block hole 734b. Referring to Figs. 13C and 13D, second anchor 814 has a flat, generally circular proximal surface 836, and a rounded distal surface 838, giving the anchor a generally hemispherical or "crescent" shape. (Alternatively, surface 836 can be concave.) Anchor 814 defines three through-holes, 830, 834a, and 834b. The holes pass in an axial direction from the anchor's proximal circular surface 836 to its distal rounded surface 838.

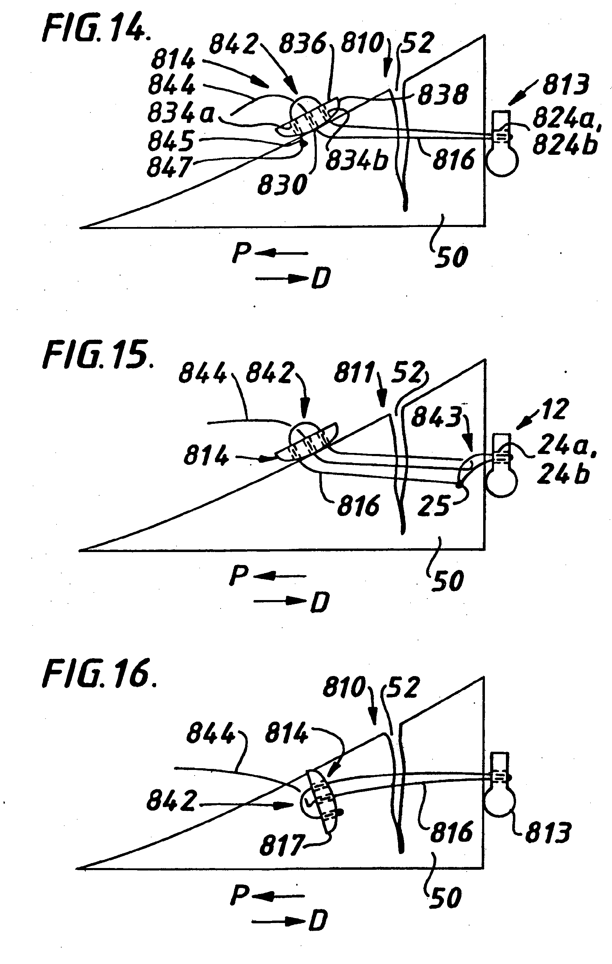

Fig. 14 illustrates deployment of a device 810 that has a crescent-shaped second anchor 814, and a "pulley" anchor 813. Pulley anchor 813 does not act as a dead-end for a suture, nor does it include a one-way knot. Instead, pulley anchor 813 includes two through-holes, 824a and 824b. A suture 816 passes through hole 824a in a first direction, and then through hole 824b in a second direction, such that suture 816 can slide over pulley anchor 813 in either direction. Pulley anchor 813 can have the shape and structure of anchor 12 (i.e., the holes are located on a fin), the simple cylindrical structure of anchor 112 of Fig. 7A, or numerous other structures.

In operation, anchors 813 and 814 are deployed using, e.g., a hollow needle 70, such that anchor 814 is positioned on a proximal side of meniscus 50, and anchor 813 is deployed against distal surface 58. When deployed, suture 816 passes through hole 834a of crescent-shaped anchor 814 in a generally proximal direction (arrow P), from rounded surface 838 to circular surface 836, and then through hole 834b in a generally distal direction (arrow D), forming a loop 842. From loop 842, the suture passes through meniscal tissue, through hole 824a of pulley anchor 813, through hole 824b of anchor 813, and back through meniscal tissue to the crescent shaped anchor. The suture then passes through loop 842, terminating at free end 844. The opposite end 845 of suture 816 includes a knob or a knot 847 that prevents end 845 from passing through hole 834a. Thus, suture 816 begins at anchor 814, in addition to forming the one-way knot at anchor 814.

Referring to Fig. 15, rather than beginning at anchor 814, the suture can be affixed to a first anchor 12. In Fig. 15, a device 811 includes a first anchor 12 and crescent-shaped second anchor 814. Suture 816 passes through holes 24a and 24b of anchor 12, forming a knot 25 as shown in Fig. 1A. From knot 25, suture 816 passes through meniscal tissue and then through holes 834a and 834b of anchor 814, forming loop 842. From loop 842, the suture passes back through meniscal tissue to first anchor 12, and then through a fixed loop 843 located between knot 25 and first anchor 12. The suture then passes back through meniscal tissue, through hole 830 of anchor 814, and through loop 842, terminating at free end 844.

When a surgeon pulls on free end 844 in device 811, the mechanical advantage is 3:1, since suture 816 passes between the two anchors three times. By comparison, in device 810, the mechanical advantage is 2:1.

Referring to Fig. 16, the crescent shaped anchor 814 of device 810 can be deployed inter-body (i.e., embedded within meniscal tissue), rather than against a surface of the meniscus. In this deployment method, device 810 is deployed in the manner described above with reference to Fig. 14, or using another deployment method. After positioning the anchors and tensioning free end 844, however, the surgeon pushes anchor 814 into the meniscal tissue, using, e.g., a needle. To facilitate pushing anchor 814 into the tissue, a point 817 of anchor 814 can be sharp.

Other types of second anchors described herein, whether or not they include a sharp point, can also be positioned inter-body.

In each of the described embodiments of the second anchor, the one-way knot can be left "loose" until after both the first and second anchors are positioned against the meniscus. In such an embodiment, the suture would be very long, e.g., more than 12 cm long, such that the one-way knot includes considerable slack, and the loop portion of the knot is accessible to the surgeon's fingers. In this embodiment, the surgeon can position the first and second anchors against backside 58 by pulling on both the free end and the loop itself. Once the anchors are satisfactorily positioned, the surgeon tightens the knot by pulling on the free end.

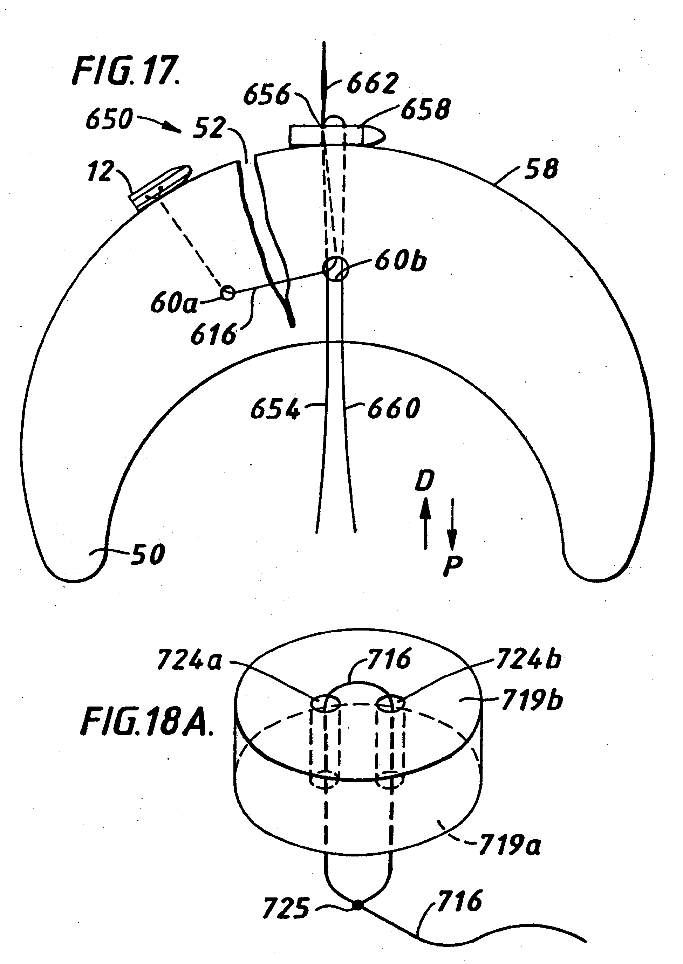

The second anchor can employ one-way tightening schemes other than a "one-way knot." For example, referring to Fig. 17, a device 650 includes first anchor 12, a second anchor 652, and two sutures 616 and 654. Second anchor 652 has a generally cylindrical shape, and defines two through-holes 656 and 658. When anchors 12 and 652 are positioned against backside 58 of meniscus 50, suture 616 passes from first anchor 12, through holes 60a and 60b in the meniscus, and then through holes 656 and 658 of anchor 652, terminating at free end 660. Suture 616 does not form a one-way knot at second anchor 652. Instead, suture 616 simply passes through holes 656 and 658 in succession, such that anchor 652 acts as a pulley anchor.

The second suture, suture 654, passes only through hole 656 of anchor 652, and through hole 60b of the meniscus. A portion 662 of suture 654, distal to hole 656, is thicker than the remainder of suture 654. This thicker portion 662 cannot pass through hole 656. (The thickness of portion 662 is exaggerated in Fig. 17.)

In operation, a surgeon deploys the two anchors as described above with respect to other embodiments, and then pulls on free end 660 of suture 616 to position the anchors against backside 58 and close the tear in the meniscus. Once suture 616 is tensioned to the surgeon's satisfaction, the surgeon pulls on suture 654 in the proximal direction (arrow P), until a segment of portion 662 wedges into hole 656. Portion 662 wedges suture 616 in place within hole 656, preventing the length of suture 616 between the two anchors from increasing, and thereby locking the two anchors in place.

Modifications of other portions of wound closure device 10 are also possible. For example, the fin-shaped projection 20 of the first anchor need not have the shape shown in the figures. Other types of projections capable of protruding through a needle opening and engaging tissue can be used. In addition, as described above with reference to Figs. 6A, the first anchor need not include any projection, but can instead be a simple cylinder defining holes for affixation of the suture.

Instead of attaching the suture to the first anchor using a conventional knot 25, the suture can be welded or glued to the anchor, or can be spliced.

Referring to Fig. 18A, the first anchor need not include an extended, cylindrical body, but can instead have a button-shaped body. Button-shaped first anchor 712 includes a circular distal side 719a and a circular proximal side 719b. Two axial holes 724a and 724b pass from side 719a to side 719b. A suture 716 is attached to anchor 712 by passing through hole 724a in a first direction, through hole 724b in a second direction, and then forming a conventional knot 725 on the distal side of the anchor.

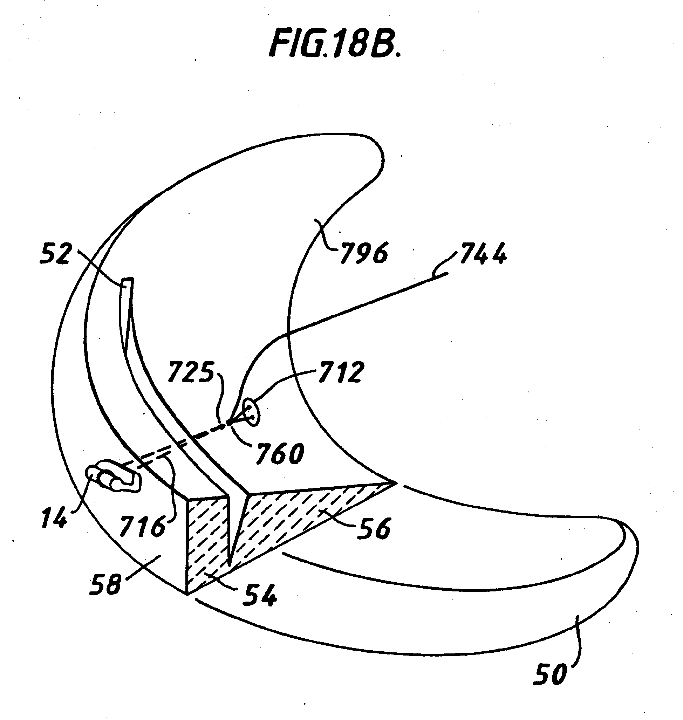

Referring to Fig. 18B, button-shaped first anchor 712 is deployed against a proximal surface 796 of meniscus 50, and second anchor 14 is deployed against surface 58. Suture 716 passes from first anchor 712 into proximal tissue 56 at point 760, such that knot 725 is located within the tissue. From point 760, suture 716 passes across tear 52 to second anchor 14, then through second anchor 14 in the manner described above with reference to Fig. 1. From second anchor 14, the suture passes back into distal tissue 54, across tear 52, and emerges from proximal tissue 56 at point 760. The suture then terminates at free end 744. As with the embodiments described above, pulling on free end 744 tensions the suture and closes the tear.

Alternatively, button-shaped anchor 712 can be deployed on the distal side of the tear adjacent second anchor 14, using the methods described above with reference to Figs. 2, 5A-5D, 6A-6D, and 7A-7D.

The second anchor need not include a groove 35 to allow passage of a plunger. Instead, the second anchor can define an axial through-hole for passage of the plunger.

Rather than a suture, the first and second anchors can be connected with other types of flexible members.

The wound closure device can include more than two suture anchors. For example, in addition to the first and second anchors, the device can include a third anchor identical in structure and function to the second anchor. In operation, after deploying the second anchor against surface 58 of meniscus 50, the surgeon could again pass the suture across tear 52, adding an additional stitch, and then deploy the third anchor against surface 58. After deploying all three anchors, the surgeon would pull on the free end of the suture, causing the suture to slide over both the second and third anchors, shortening the length of suture between the third and first anchors, and thereby closing the wound.

When more than two anchors are used, one or more of the anchors can be a pulley, such as pulley anchor 813 described above with reference to Fig. 14. For example, the device could include a first anchor 12, a pulley anchor 813, and a second anchor 14. The suture would be affixed to the first anchor, would slide over the pulley anchor, and form a one-way knot at the second anchor.

Depending on the size of the tissue wound, more than three anchors can be used. The additional anchors can all be pulley anchors, can all be similar to the second anchor, or can be include both additional pulleys and additional second anchors.

The wound closure devices need not be deployed using a needle, and need not be deployed arthroscopically. Instead, a surgeon can place the anchors against the tissue during an open procedure.

The wound closure devices can be used to repair tissue wounds other than meniscal tears. For example, the devices can be used to repair tears in skin, muscles, and ligaments, and to re-attach tissue to support structures, such as bones.

## Claims

1. Wound closure device comprising: a first anchor (12); a second anchor (14); a flexible member (16) connecting the first anchor (12) to the second anchor (14), the flexible member (16) having a free end (44) and being movably attached to the second anchor (14), wherein the movable attachment enables the length of the flexible member (16) between the first (12) and second (14) anchors to be shortened, but not lengthened and includes a knot (40) formed in the flexible member at the second anchor (14), wherein the knot (40) includes a first portion of the flexible member (16) that forms a loop (42), and a second portion that passes over a surface of the second anchor and through the loop (42), such that pulling on the free end (44) of the flexible member (16) shortens the length of the flexible member (16) between the first (12) and second (14) anchors, wherein the first anchor (12) comprises a generally cylindrical body, the body being sized and shaped to fit within a bore of a hollow needle, and wherein the second anchor comprises a distal surface (29a), a proximal surface (29b) **characterized in that** the second anchor further comprises a generally cylindrical body (28) and an appendage attached to the generally cylindrical body (28), there being a plurality of holes (30,34a, 34b) passing through the second anchor.

2. Wound closure device according to claims 1, wherein the first anchor (12) comprises a projection extending from the cylindrical body.

3. Wound closure device according to claim 2, wherein the projection comprises a longitudinal fin (20).

4. Wound closure device according to any one of claims 1 to 3, wherein the generally cylindrical body (28) and the appendage define an interior, partially enclosed region (39).

5. Wound closure device according to any one of claims 1 to 4, wherein the distal surface (29a) is bevelled.

6. Wound closure device according to any one of claims 1 to 5, wherein the appendage comprises a stem (236).

7. Wound closure device according to any one of claims 1 to 5, wherein the appendage is generally rectangular in cross section.

8. Wound closure device according to claim 7, wherein the appendage (426) is attached to the generally cylindrical body (428) along the long side of the rectangle and is centered along an axial length of the generally cylindrical body (428).

9. Wound closure device according to any preceding claim, wherein the surface over which the second portion of the flexible member passes comprises an exterior surface (41) of the second anchor (14).

10. Wound closure device according to claim 9, wherein the knot is configured such that pulling on the free end (44) causes the flexible member (16) to slide through the loop (42) to shorten the length of the flexible member between the first (12) and second (14) anchors, but pulling on the flexible member (16) in an opposite direction in order to increase the length of the flexible member (16) between the first (12) and second (14) anchors causes the loop (42) to press the second portion against a compression surface (43) of the second anchor (14), resisting increase in the length of the flexible member (16) between the first (12) and second (14) anchors.

11. Wound closure device according to any one of claims 9 or 10, wherein the second portion includes the free end (44).

12. Wound closure device according to any preceding claim, wherein the second anchor (14) includes at least two loop-forming holes (34a, 34b) and the first portion of the flexible member (16) passes through the at least two loop-forming holes (34a, 34b) to form the loop (42).

13. Wound closure device according to claim 2, wherein the projection defines at least one hole (24a, 24b) for passage of the flexible member (16) therethrough.

14. Wound closure device according to any one of the preceding claims, wherein the flexible member (16) comprises a suture.

## Patentansprüche

1. Eine Vorrichtung zum Verschließen einer Wunde, beinhaltend: einen ersten Anker (12); einen zweiten Anker (14); ein biegsames Element (16), das den ersten Anker (12) mit dem zweiten Anker (14) verbindet, wobei das biegsame Element (16) ein freies Ende (44) aufweist und an dem zweiten Anker (14) beweglich angebracht ist, wobei die bewegliche Anbringung ermöglicht, dass die Länge des biegsamen Elements (16) zwischen dem ersten (12) und dem zweiten (14) Anker verkürzt, aber nicht verlängert wird, und einen Knoten (40) umfasst, der in dem biegsamen Element an dem zweiten Anker (14) gebildet ist, wobei der Knoten (40) einen ersten Abschnitt des biegsamen Elements (16), der eine Schlaufe (42) bildet, und einen zweiten Abschnitt, der über eine Oberfläche des zweiten Ankers und durch die Schlaufe (42) verläuft, umfasst, so dass das Ziehen an dem freien Ende (44) des biegsamen Elements (16) die Länge des biegsamen Elements (16) zwischen dem ersten (12) und dem zweiten (14) Anker verkürzt, wobei der erste Anker (12) einen im Allgemeinen zylindrischen Körper beinhaltet, wobei der Körper bemessen und geformt ist, um in eine Bohrung einer Hohlnadel zu passen, und wobei der zweite Anker eine distale Oberfläche (29a), eine proximale Oberfläche (29b) beinhaltet, **dadurch gekennzeichnet, dass** der zweite Anker ferner einen im Allgemeinen zylindrischen Körper (28) und einen Fortsatz, welcher an dem im Allgemeinen zylindrischen Körper (28) angebracht ist, beinhaltet, wobei eine Vielzahl von Löchern (30, 34a, 34b) vorhanden ist, die durch den zweiten Anker verlaufen.

2. Vorrichtung zum Verschließen einer Wunde gemäß Anspruch 1, wobei der erste Anker (12) einen Vorsprung beinhaltet, der sich von dem zylindrischen Körper erstreckt.

3. Vorrichtung zum Verschließen einer Wunde gemäß Anspruch 2, wobei der Vorsprung eine Längsrippe (20) beinhaltet.

4. Vorrichtung zum Verschließen einer Wunde gemäß einem der Ansprüche 1 bis 3, wobei der im Allgemeinen zylindrische Körper (28) und der Fortsatz einen inneren, teilweise umschlossenen Bereich (39) definieren.

5. Vorrichtung zum Verschließen einer Wunde gemäß einem der Ansprüche 1 bis 4, wobei die distale Oberfläche (29a) abgeschrägt ist.

6. Vorrichtung zum Verschließen einer Wunde gemäß einem der Ansprüche 1 bis 5, wobei der Fortsatz einen Stamm (236) beinhaltet.

7. Vorrichtung zum Verschließen einer Wunde gemäß einem der Ansprüche 1 bis 5, wobei der Fortsatz im Querschnitt im Allgemeinen rechteckig ist.

8. Vorrichtung zum Verschließen einer Wunde gemäß Anspruch 7, wobei der Fortsatz (426) entlang der langen Seite des Rechtecks an dem im Allgemeinen zylindrischen Körper (428) angebracht und entlang einer axialen Länge des im Allgemeinen zylindrischen Körpers (428) zentriert ist.

9. Vorrichtung zum Verschließen einer Wunde gemäß einem der vorhergehenden Ansprüche, wobei die Oberfläche, über die der zweite Abschnitt des biegsamen Elements verläuft, eine äußere Oberfläche (41) des zweiten Ankers (14) beinhaltet.

10. Vorrichtung zum Verschließen einer Wunde gemäß Anspruch 9, wobei der Knoten so konfiguriert ist, dass das Ziehen an dem freien Ende (44) bewirkt, dass das biegsame Element (16) durch die Schlaufe (42) gleitet, um die Länge des biegsamen Elements zwischen dem ersten (12) und dem zweiten (14) Anker zu verkürzen, aber das Ziehen an dem biegsamen Element (16) in eine entgegengesetzte Richtung, um die Länge des biegsamen Elements (16) zwischen dem ersten (12) und dem zweiten (14) Anker zu vergrößern, bewirkt, dass die Schlaufe (42) den zweiten Abschnitt gegen eine Kompressionsoberfläche (43) des zweiten Ankers (14) drückt, was einer Vergrößerung der Länge des biegsamen Elements (16) zwischen dem ersten (12) und dem zweiten (14) Anker Widerstand bietet.

11. Vorrichtung zum Verschließen einer Wunde gemäß einem der Ansprüche 9 oder 10, wobei der zweite Abschnitt das freie Ende (44) umfasst.

12. Vorrichtung zum Verschließen einer Wunde gemäß einem der vorhergehenden Ansprüche, wobei der zweite Anker (14) mindestens zwei Schlaufen bildende Löcher (34a, 34b) umfasst und der erste Abschnitt des biegsamen Elements (16) durch die mindestens zwei Schlaufen bildenden Löcher (34a, 34b) verläuft, um die Schlaufe (42) zu bilden.

13. Vorrichtung zum Verschließen einer Wunde gemäß Anspruch 2, wobei der Vorsprung mindestens ein Loch (24a, 24b) für das Verlaufen des biegsamen Elements (16) dort hindurch definiert.

14. Vorrichtung zum Verschließen einer Wunde gemäß einem der vorhergehenden Ansprüche, wobei das biegsame Element (16) ein Nahtmaterial beinhaltet.

## Revendications

1. Dispositif de fermeture de plaie comprenant : une première ancre (12) ; une deuxième ancre (14) ; un élément flexible (16) raccordant la première ancre (12) à la deuxième ancre (14), l'élément flexible (16) ayant une extrémité libre (44) et étant attaché de façon mobile à la deuxième ancre (14), où l'attache mobile permet à la longueur de l'élément flexible (16) entre les première (12) et deuxième (14) ancres d'être raccourcie, mais pas allongée et inclut un noeud (40) formé dans l'élément flexible au niveau de la deuxième ancre (14), où le noeud (40) inclut une première portion de l'élément flexible (16) qui forme une boucle (42), et une deuxième portion qui passe par-dessus une surface de la deuxième ancre et à travers la boucle (42), de telle manière que le fait de tirer sur l'extrémité libre (44) de l'élément flexible (16) raccourcit la longueur de l'élément flexible (16) entre les première (12) et deuxième (14) ancres, où la première ancre (12) comprend un corps généralement cylindrique, le corps étant dimensionné et conformé afin de s'emboîter au sein d'un alésage d'une aiguille creuse, et où la deuxième ancre comprend une surface distale (29a), une surface proximale (29b) **caractérisé en ce que** la deuxième ancre comprend en sus un corps généralement cylindrique (28) et un appendice attaché au corps généralement cylindrique (28), la deuxième ancre étant traversée par une pluralité de trous (30, 34a, 34b).

2. Dispositif de fermeture de plaie selon la revendication 1, où la première ancre (12) comprend une projection s'étendant à partir du corps cylindrique.

3. Dispositif de fermeture de plaie selon la revendication 2, où la projection comprend une ailette longitudinale (20).

4. Dispositif de fermeture de plaie selon l'une quelconque des revendications 1 à 3, où le corps généralement cylindrique (28) et l'appendice définissent une région intérieure partiellement enclose (39).

5. Dispositif de fermeture de plaie selon l'une quelconque des revendications 1 à 4, où la surface distale (29a) est biseautée.

6. Dispositif de fermeture de plaie selon l'une quelconque des revendications 1 à 5, où l'appendice comprend une tige (236).

7. Dispositif de fermeture de plaie selon l'une quelconque des revendications 1 à 5, où l'appendice est de coupe transversale généralement rectangulaire.

8. Dispositif de fermeture de plaie selon la revendication 7, où l'appendice (426) est attaché au corps généralement cylindrique (428) le long du côté long du rectangle et est centré le long d'une longueur axiale du corps généralement cylindrique (428).

9. Dispositif de fermeture de plaie selon n'importe quelle revendication précédente, où la surface par-dessus laquelle passe la deuxième portion de l'élément flexible comprend une surface extérieure (41) de la deuxième ancre (14).

10. Dispositif de fermeture de plaie selon la revendication 9, où le noeud est configuré de telle manière que le fait de tirer sur l'extrémité libre (44) amène l'élément flexible (16) à glisser à travers la boucle (42) afin de raccourcir la longueur de l'élément flexible entre les première (12) et deuxième (14) ancres, mais le fait de tirer sur l'élément flexible (16) dans une direction opposée afin d'accroître la longueur de l'élément flexible (16) entre les première (12) et deuxième (14) ancres amène la boucle (42) à presser la deuxième portion contre une surface de compression (43) de la deuxième ancre (14), opposant une résistance à l'accroissement de la longueur de l'élément flexible (16) entre les première (12) et deuxième (14) ancres.

11. Dispositif de fermeture de plaie selon l'une quelconque des revendications 9 ou 10, où la deuxième portion inclut l'extrémité libre (44).

12. Dispositif de fermeture de plaie selon n'importe quelle revendication précédente, où la deuxième ancre (14) inclut au moins deux trous de formation de boucle (34a, 34b) et la première portion de l'élément flexible (16) passe à travers ces au moins deux trous de formation de boucle (34a, 34b) afin de former la boucle (42).

13. Dispositif de fermeture de plaie selon la revendication 2, où la projection définit au moins un trou (24a, 24b) pour que l'élément flexible (16) passe à travers.

14. Dispositif de fermeture de plaie selon l'une quelconque des revendications précédentes, où l'élément flexible (16) comprend un fil de suture.
